# EUROPEAN PATENT APPLICATION

(11) **EP 1 698 349 A1**
(43) Date of publication of application: **06.09.2006**
(21) Application number: 04808117.8
(22) Date of filing: 24.12.2004
(51) Int. Cl.: A61K 47/22, A61K 9/16, A61K 9/20

(54) **METHOD OF IMPROVING SUITABILITY FOR GRANULATION**

(30) Priority: 25.12.2003 JP 2003430169
(71) Applicant: Takeda Pharmaceutical Company Limited, Osaka-shi, Osaka 541-0045 (JP)
(72) Inventor: MURAKAWA, Yusuke c/o Takeda Pharma Co. Ltd., Osaka-shi, Osaka 532-8686 (JP); FUKUTA, Makoto c/o Takeda Pharma, Co, Ltd.,, Osaka-shi, Osaka 532-8686 (JP)
(74) Representative: Rickard, Timothy Mark Adrian
(86) International application number: PCT/JP2004/019767
(87) International publication number: WO 2005/065715

(57) **Abstract**

The present invention provides a granulated product having improved granulatability which contains a compound with poor wettability and a surfactant, particularly the granulated product wherein at least about 35% by weight with respect to the total weight of the product does not pass through a 100-mesh sieve, and a method for preparing the granulated product having improved granulatability containing a compound with poor wettability, which comprises adding a surfactant to the compound before or during the granulation, particularly at a weight ratio of about 0.001 to about 2.

## Description

### Technical Field

The present invention relates to a solid composition, in particular a solid pharmaceutical composition, having improved granulatability, and a method for preparation thereof.

### Background Art

Many of general tablets or capsules as oral dosage forms are often first made as granulated products, and then directed to a compression molding process or a capsule filling process, in order to improve the handling of the powders or the properties of preparation in the preparation process. In particular, if the active compounds are contained in a high proportion, it is considered preferable to carry out granulation for the purpose of avoiding troubles during the preparation process. Examples of the granulation process include a wet process, a dry process, a spray granulation process, or the like. However, wet granulation is widely employed in terms of the modification of the surface properties of the active compound or the efficiency of preparation, among which a fluidized bed granulation process is predominantly used. This process facilitates the particle growth by the repetition of a series of the processes for continuously spraying a binder liquid of hydroxypropyl cellulose, povidone, or the like on the granulation components, and then drying the resultant product.

However, when the formulations which contain the compound having poor wettability (hereinafter, sometimes referred to "low water-soluble" which is interchangeably used with "poor wettability" in the present specification unless otherwise particularly mentioned for convenience purpose) as an active ingredient are granulated in a fluidized bed, granulation may not proceed due to the poor wettability of the active ingredient to the binder. In such case, low water-soluble components are usually diluted with other excipients to low concentrations so as to improve granulatability, but in the case of the formulation containing high proportions of the low water-soluble components, this method leads to enlarged size of the tablets (or capsules), thus causing problems in compliance.

### Disclosure of the Invention

Therefore, it is an object of the present invention to provide a novel methods for improving granulatability which can be desirably applied to a solid composition containing a high proportion of the low water-soluble compounds; and thus to provide a solid composition, in particular a solid pharmaceutical composition, containing a high proportion of the low water-soluble compounds, which has a solid size allowing the compliance to be not adversely affected, and has improved granulatability.

The present inventors have extensively studied in order to solve the above-described problems and have found that a granulated product having suitable particles sizes, bulk/fillability, and flowability with solid sizes in the ranges suitable for the applications, can be prepared even in the case of the formulations having a high proportion of the low water-soluble compounds, by blending a small amount of surfactants into a binder solution. On a basis of these findings, the present inventors have further made studies, and thus have reached the completion of the present invention.

Specifically, the present invention provides:
[1] a pharmaceutical granulated product having improved granulatability, which contains a pharmaceutical compound with poor wettability and a surfactant,
[2] a granulated product, wherein the product contains a compound with poor wettability and a surfactant, and at least about 35% by weight with respect to the total weight of the product does not pass through a 100-mesh sieve,
[3] the granulated product as described in the above [2], wherein the weight ratio of the compound and the surfactant is 1 : about 0.001 to about 2,
[4] the granulated product as described in the above [3], wherein the weight ratio is 1 : about 0.001 to less than 1,
[5] the granulated product as described in the above [3], wherein the weight ratio is 1 : about 0.001 to less than 0.1,
[6] the granulated product as described in the above [3], wherein the weight ratio is 1 : about 0.005 to about 0.05,
[7] the granulated product as described in the above [2], wherein the ratio of the compound with respect to the total granulated product is about 20% by weight or more,
[8] the granulated product as described in the above [2], wherein the compound is a pharmaceutical compound,
[9] a molded product made by molding the granulated product as described in any one of the above [2] to [8],
[10] a method for improving granulatability of a pharmaceutical composition containing a pharmaceutical compound with poor wettability, which comprises adding a surfactant before or during the granulation,
[11] a method for preparing a granulated product containing a compound with poor wettability, having improved granulatability, which comprises adding a surfactant in the weight ratio of about 0.001 to about 2 with respect to the compound before or during the granulation,
[12] the method as described in the above [11], wherein a granulated product is obtained in which at least about 35% by weight with respect to the total weight of the product does not pass through a 100-mesh sieve,
[13] the method as described in the above [11], which involves wet granulation in a binder solution containing a surfactant,
[14] the method as described in the above [13], wherein the concentration of the surfactant in the binder solution is about 1 to about 1,000 mmol/L,
[15] the method as described in the above [13], wherein the concentration of the surfactant in the binder solution is about 10 to about 100 mmol/L,
[16] the method as described in the above [11], wherein the compound is a pharmaceutical compound,
[17] a method for preparing a molded product, comprising molding the granulated product which is obtained by the method as described in any one of the above [11] to [16],
[18] a use of a surfactant for improving granulatability during the granulation of a pharmaceutical composition, and
[19] an agent for improving granulatability during the granulation of a pharmaceutical composition, which contains a surfactant.

The preparation method of the present invention improves wettability of the low water-soluble components to a binder solution by adding a small amount of a surfactant before or during the granulation, and thus it exhibits excellent effects that a solid composition having improved granulatability can be provided without blending a large amount of an excipient.

### Best Mode for Carrying out the Invention

The pharmaceutical composition of the present invention is not particularly limited as long as it contains a pharmaceutical compound with poor wettability and a surfactant, and has improved granulatability by the action of the surfactant. As used herein, the "pharmaceutical compound" means any compound having physiological activity which can be used as a medicine. When the pharmaceutical compound of the present invention has poor wettability, it may be either water-soluble or low water-soluble (herein the "low water-soluble" has the meaning as usually used in the art, that is, it means low soluble in water, and specifically, for example, it may be "poorly soluble", "sparingly soluble" or "hardly soluble" in water according to the definition in the Japanese Pharmacopoeia (i.e., the dissolution rate in water is 10000 ppm or less, and preferably 100 ppm or less within 30 minutes after mixing under shaking for 30 seconds every 5 minute at 20±5°C, or the solubility in water is 10 mg/mL or less, and preferably 0.1 mg/mL or less under the same conditions). The pharmaceutical compound with poor wettability includes, for example, those which can be used as a medicine among the compounds exemplified as the granulated product of the present invention as described below.

The content of the pharmaceutical compound in the pharmaceutical composition of the present invention varies depending on the formulation, the administration route, the carrier, or the like, but the present invention is particularly suitable for the formulation having the ranges which are preferably exemplified as the weight ratio of the low water-soluble compound with respect to the total granulated product for the granulated product of the present invention as described below, that is, the formulation having a relatively high proportion of the pharmaceutical compound.

As used in the present specification, the expression "improved granulatability" means having the particle sizes and flowability which are suitable for the purpose of reduction of adhesion, scattering or flocculation of the fine powders of the pharmaceutical compound, easy dosing, easy weighing, or the like for (granulated) powdered drugs, granules, fine granules, or the like, and further improvement of the compression or fillability for tablets, capsules or the like; or the like, and specifically it can be examined by measuring the particle size distribution, the specific volume and the angle of repose of the granulated product. For example, the particle size distribution and the angle of repose within the range indicated as the characteristics of the granulated product of the present invention as described below are preferable.

The surfactant used in the pharmaceutical composition of the present invention is not particularly limited as long as it has sufficient wettability-improving action so as to improve the granulatability of the pharmaceutical composition in a small amount, and it may likewise include those exemplified for the granulated product of the present invention as described below.

The blending ratio of the pharmaceutical compound and the surfactant in the pharmaceutical composition according to the present invention is not particularly limited as long as it is within the range capable of improving granulatability of the pharmaceutical composition, but it is preferably exemplified by the range depicted as the weight ratio of the low water-soluble compounds and the surfactant for the granulated product of the present invention as described below.

The pharmaceutical composition of the present invention may further contain a diluent, a lubricant, a binder, a disintegrant, a coating agent, and the like, in addition to the pharmaceutical compound and the surfactant. Furthermore, if necessary, formulation additives such as a preservative, an antioxidant, a colorant, a sweetening agent, and the like can be contained. These formulation additives may like include those exemplified for the granulated product of the present invention as described below.

The formulation of the pharmaceutical composition of the present invention may include any one which involves a granulation process for the preparation processes, but it includes, for example, (granulate) powdered drugs, granules, fine granules, tablets, capsules, suppositories, pills, and the like.

The pharmaceutical composition of the present invention can be prepared in the form of a pharmaceutical composition having improved granulatability obtained by blending a surfactant with the pharmaceutical compound before or during granulation, as it is, or if desired, a final preparation obtained by further molding the composition. Therefore, the present invention also provides a method for improving the granulatability of the pharmaceutical composition by the addition of a surfactant before or during granulation. The specific process in either the case of adding a surfactant before granulation, or the case of adding a surfactant during granulation is described in detail in a method for preparing the granulated product of the present invention as described below. For each of the processes such as milling, mixing (kneading), granulation, coating, and the like, a diluent, a lubricant, a binder, a disintegrant, a thickener, a coating agent, or the like may be added. Furthermore, if desired, the formulation additives such as a preservative, an antioxidant, a colorant, a sweetening agent, and the like can be also added in some of the above-described processes.

The present invention further provides a granulated product (hereinbelow, sometimes abbreviated as "the granulated product of the present invention"), wherein the product contains a compound with poor wettability (low water-solubility) and a surfactant, and at least about 35 % by weight of the total weight does not pass through a 100-mesh sieve [dimension of the opening of the sieve: 149 µm (average tolerance ± 6%): JIS Sieve Regulation]. It is believed that the granulated product having such degree of the particle size distribution sufficiently reduces adhesion, scattering or flocculation of the fine powders of the low water-soluble compounds, and allows easy dosing, and easy weighing, as well as improves compression moldability and capsule fillability.

The granulated product of the present invention further preferably has the characteristics of an angle of repose of 45° or less, more preferably 40° or less, and particularly preferably 35° or less. The angle of repose of the granulated product is measured by the following method.

Specifically, 20 g of a granulated product is supplied from the upper funnel using a powder tester (TYPE PT-E manufactured by Hosokawa Micron Corp.) and is subjected to moderate vibration by means of a vibrator, causing the granulated product to be dropped. After the powders dropped completely, the angle between the powder layer and the horizontal surface is referred to as an angle of repose.

As described above, the "low water-soluble compound" in the context of the present specification has the same meaning of "a compound with poor wettability" unless otherwise specifically mentioned. The "compound with poor wettability" specifically means a compound having a contact angle between the bulk molded product and water of about 30° or more. Accordingly, as used in the present specification, the expression "low water-soluble compound" does not necessarily coincide with the compound which is regarded as "hardly soluble in water", "sparingly soluble in water", or "insoluble in water" accordingly to the definition of the Japanese Pharmacopeia. The contact angle is measured by the following method.

Specifically, about 300 mg of a bulk is weighed, and compression molded to a bulk molded product using a plain mortar and pestle of 10.0 mmφ by means of an autograph (Shimadzu Corporation) under the conditions of a tableting pressure of 10 kN/pestle, and a tableting speed of 10 mm/min. On the planar surface of the bulk molded product, about 10 µL of purified water is dropped using a Microsyringe (Ito Corporation), and the solid/liquid interface immediately after the dropping is photographed by a digital microscope (Keyence Corporation). After photographing, an angle between the liquid surface and the solid surface is measured, and referred to as a contact angle.

The present invention can be preferably used for the low water-soluble compounds having a contact angle between the bulk molded product and water of about 40° or more, and in particular, the low water-soluble compounds having a contact angle between the bulk molded product and water of about 50° or more.

The low water-soluble compound is not particularly limited as long as it has the above-described characteristics, but it includes, for example, low water-soluble pharmaceutical compounds or compounds as the food components, or compounds used as an active ingredient for an animal drug, agricultural chemicals, feed, sanitary products, or the like.

The low water-soluble pharmaceutical compound includes, for example, benzoxazepine compounds as described in JP-A-2002-080468, heterocyclic compounds as described in JP-A-11-60571, low water-soluble or water-insoluble pharmaceutical compounds as described in JP-A-2002-302435, and the like.

These low water-soluble pharmaceutical compounds may be pharmaceutically acceptable salts, such as salts with inorganic bases, salts with organic bases, salts with inorganic acids, salts with organic acids, salts with basic or acidic amino acids, and the like. Preferable examples of the salts with inorganic bases include, for example, alkali metal salts such as sodium salts, potassium salts, and the like; alkaline earth metal salts such as calcium salts, magnesium salts, and the like; aluminum salts; ammonium salts; and the like. Preferred examples of the salts with organic bases include, for example, salts with trimethylamine, triethylamine, pyridine, picoline, ethanolamine, diethanolamine, triethanolamine, dicyclohexylamine, N,N'-dibenzylethylenediamine, and the like. Preferred examples of the salts with inorganic acids include, for example, salts with hydrochloric acid, hydrobromic acid, nitric acid, sulfuric acid, phosphoric acid, and the like. Preferred examples of the salts with organic acids include, for example, salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like. Preferred example of the salts with basic amino acids include, for example, salts with arginine, lysine, ornithine, or the like, and preferable examples of the salt with acidic amino acids include, for example, salts with asparagic acid, glutamic acid, or the like.

The low water-soluble pharmaceutical compound or a salt thereof can be obtained by a per se known method, for example, the methods described in each of the above-described documents and the methods analogous or equivalent thereto. In the case where the compound is obtained in the free form, it can be converted into a desired salt according to a per se known method, or the methods analogous or equivalent thereto, whereas in the case where the compound is obtained as a salt, it can be converted into the free form or the desired other salts according to a per se known method, or the methods analogous or equivalent thereto.

The compound may be hydrated or non-hydrated.

Furthermore, in the case where the compound is obtained as a mixture of optically active substances, this can be separated into a desired optically active substance by a per se known optical resolution means.

The low water-soluble pharmaceutical compound or a salt thereof may be a prodrug, that is, a compound which will be converted into the compound by the reaction using an enzyme, a gastric acid or the like in vivo under the physiological conditions. Further, the prodrug may be converted into the compounds under the physiological conditions, as described in "Development of Pharmaceuticals" published by Hirokawa Shoten, Vol. 7, "Molecular Design", pages 163-198 (1990). The prodrug can be prepared from the active compounds according to a per se known method.

The low water-soluble compounds as the food components include, for example, coenzyme Q₁₀, oil-soluble vitamins (for example, vitamin A, vitamin D, vitamin E, vitamin F, vitamin K, vitamin U, etc.), vitamin derivatives as described in JP-A-2002-302435, and the like, but are not limited thereto. These compounds may be salts which are acceptable for use in foods. Such the salts likewise include those exemplified as pharmaceutically acceptable salts of the above-described low water-soluble pharmaceutical compounds. Further, the low water-soluble compounds as the food components may be precursor compounds which can be converted to the above-described coenzyme Q₁₀, oil-soluble vitamins, vitamin derivatives, etc. in vivo.

Furthermore, the low water-soluble agrochemical compounds include, for example, low water-soluble or low soluble, solid agrochemical compounds as described in JP-A-2002-302435, and the like, but are not limited thereto. These compounds may be agrochemically acceptable salts. Such the salts likewise include those exemplified as pharmaceutically acceptable salts of the above-described low water-soluble pharmaceutical compounds. Further, low water-soluble agrochemical compounds may be precursor compounds which can be converted into the agrochemical compounds in vivo in target organisms (pests, weeds, etc.).

The surfactants used in the granulated product of the present invention are not particularly limited as long as they have wettability-improving action, sufficient to improve the granulatability of the pharmaceutical composition in a small amount, and for example, non-ionic surfactants, anionic surfactants, cationic surfactants, amphoteric surfactants, naturally-derived surfactants, or the like are used.

As the non-ionic surfactants, higher alcohol-ethylene oxide adducts, alkylphenol-ethylene oxide adducts, aliphatic acid-ethylene oxide adducts, polyhydric alcohol fatty acid ester-ethylene oxide adducts, higher alkylamine-ethylene oxide adducts, aliphatic acid amide-ethylene oxide adducts, fat and oil-ethylene oxide adducts, glycerin fatty acid esters, fatty acid esters of pentaerythritol, alkyl ethers of polyhydric alcohols, aliphatic acid amides of alkanolamines, or the like are used.

Of the nonionic surfactants, fatty acid esters of sorbitol and sorbitan, polyoxyethylene sorbitan fatty acid ester, sucrose fatty acid ester, polyethylene glycol, polyethylene glycol fatty acid esters, polyoxyethylene castor oil, polyoxyethylene hydrogenated castor oil, polyoxyethylene-polypropylene glycol copolymers, glycerin fatty acid esters, polyglycerol fatty acid esters, or the like are preferably used. As the sorbitan fatty acid esters, sorbitan monostearate (trade name: SS-10, Nikko Chemicals Co., Ltd.), sorbitan sesquioleate (trade name: SO-15, Nikko Chemicals Co., Ltd.), sorbitan trioleate (trade name: SO-30, Nikko Chemicals Co., Ltd.), or the like is particularly preferred. As the polyoxyethylene sorbitan fatty acid esters, polysorbate 20 (trade name: TL-10, Nikko Chemicals Co., Ltd.), 40 (trade name: TP-10, Nikko Chemicals Co., Ltd.), 60 (trade name: TS-10, Nikko Chemicals Co., Ltd.), 80 (trade name: TO-10, Nikko Chemicals Co., Ltd.), or the like are particularly preferred. As the sucrose fatty acid esters, sucrose palmitate (for example, trade name: P-1670, Mitsubishi-Kagaku Foods Corporation), sucrose stearic acid esters (for example, trade name: S-1670, Mitsubishi-Kagaku Foods Corporation), or the like are particularly preferred. As the polyethylene glycol, polyethylene glycol 200, polyethylene glycol 300, polyethylene glycol 400, polyethylene glycol 600, or the like are preferred. As the polyethylene glycol fatty acid esters, polyethylene glycol monolaurate (10 E.O.) (trade name: MYL-10, Nikko Chemicals Co., Ltd.), or the like are particularly preferred. As the polyoxyethylene castor oil, polyoxyethylene glycerol trirecinolate 35 (Polyoxy 35 Castor Oil, trade name: Cremophor EL or EL-P, BASF Japan Ltd.), or the like are particularly preferred. As the polyoxyethylene hydrogenated castor oil, Polyoxyethylene Hydrogenated Castor Oil 50, Polyoxyethylene Hydrogenated Castor Oil 60, or the like are particularly preferred. As the polyoxyethylene-polyoxypropylene glycol copolymers, polyoxyethylene (160) polyoxypropylene (30) glycol (trade name: Adeka Pluronic F-68, Asahi Denka Kogyo K. K.), or the like are particularly preferred. As the glycerin fatty acid esters, glyceryl monostearate (MGS series, Nikko Chemicals Co., Ltd.), or the like are preferred. As the polyglycerol fatty acid esters, monostearate tetraglycerin (MS-310, Sakamoto Yakuhin Kogyo Co., Ltd.) monolaurate decaglycerin (Decaglyn 1-L, Nikko Chemicals Co., Ltd.), or the like are particularly preferred.

As the anionic surfactants, for example, sulfuric acid esters (for example, higher alcohol sulfuric acid ester salts, higher alkyl ether sulfuric acid ester salts, sulfated oils, sulfated fatty acid esters, sulfated aliphatic acids, sulfated olefins), sulfonates (for example, sodium alkylbenzene sulfonates, oil-soluble alkylbenzene sulfonates, α-olefin sulfonates, Igepon T type, Aerosol OT type), phosphates (for example, phosphate salts of higher alcohol-ethylene oxide adduct), dithiophosphoric acid ester salts, and the like are used.

Of the anionic surfactant, for example, alkyl sulfuric acid salts such as sodium lauryl sulfate, and the like; bile acid salts such as sodium glycocholate, sodium deoxycholate, and the like; fatty acids or salts thereof such as sodium caprate or stearate; and the like are preferred.

As the cationic surfactant, for example, amine salt type cationic surfactants (for example, amine salt type cationic surfactants made from higher alkylamines, and amine salt type cationic surfactants made from lower or higher alkylaminenes), quaternary ammonium salt type cationic surfactants (for example, quaternary ammonium salt type cationic surfactants made from higher alkylamines, quaternary ammonium salt type surfactants made from lower or higher alkylamines), or the like are used.

As the amphoteric surfactants, for example, amino acid type amphoteric surfactants, betaine type amphoteric surfactants, or the like are used.

As the naturally-derived surfactants, for example, lecithin phosphatides such as egg yolk lecithin (trade name: PL-100H, Q.P. Co., Ltd.), soybean lecithin (trade name: Lecinol S-10, Nikko Chemicals Co., Ltd.), or the like are used.

Among the above-described surfactants, anionic surfactants are more preferred, and among them, sodium lauryl sulfate is particularly preferred.

Otherwise, alkyl sulfates, polysorbates, sucrose fatty acid esters (preferably those having high HLB values), polyethylene glycols, or polyoxyethylene-polyoxypropylene glycol copolymers are preferred, and alkyl sulfates or polysorbates are more preferred. Further, among the alkyl sulfates, sodium lauryl sulfate is more preferred, and among the polysorbates, polysorbate 80 is more preferred.

The weight ratio of the low water-soluble compounds and the surfactant in the granulated product of the present invention is not particularly limited as long as it is within the range such that the above characteristics of the granulated product (that is, at least about 35 % by weight of the total weight does not pass through a 100-mesh sieve) can be provided, and the unit amount of the solid composition used as a final product is suitably sized, but it is preferably 1 : about 0.001 to about 2, more preferably 1 : about 0.001 to less than 1, even more preferably 1 : about 0.001 to less than 0.1, and particularly preferably 1 : about 0.005 to about 0.05. If the amount of the surfactant relative to the compound is too low, sufficient wettability improving effect is not obtained, whereas if the amount of the surfactant relative to the compound is too high, adhesion of the obtained composition to the machine and the flocculation of the composition are increased, thus operability being degraded. Further, the moldability of the composition is also lowered. Improvement of the granulatability in the context of the present invention has the meanings, simply including improving the particle growth by improving the wettability of the compound to a binder, as well as easy handling in the processes of granulation, molding, packaging and use, and improving any other physical properties of particles, for example, reduction of adhesion, scattering or flocculation, easy dosing, easy weighing, etc. which may improve the qualities of the solid composition; further improving the compression or fillability when molded into tablets, capsules or the like.

The content of the low water-soluble compounds in the granulated product of the present invention is not particularly limited as long as the amount of the compound per the unit amount of the solid composition used as a final product is sufficient to achieve the purpose of use, and the present invention is particularly suitable for the granulation of a solid composition having a high proportion of the low water-soluble compounds. Accordingly, the content of the low water-soluble compounds is preferably about 20% by weight or more, more preferably about 30 to about 90% by weight, and particularly preferably about 40 to about 80% by weight, relative to the total granulated product.

The granulated product of the present invention may further contain an excipient, a lubricant, a binder, a disintegrant, a thickener, a coating agent, and the like, in addition to the low water-soluble compounds and the surfactant. Furthermore, if necessary, the additives such as a preservative, an antioxidant, a colorant, a sweetening agent, and the like can be contained.

Preferred examples of the excipient include, but not limited to, lactose, saccharose, glucose, maltose, corn starch, wheat flour starch, mannitol, xylitol, sorbitol, maltitol, erythritol, lactitol, paratinit, crystalline cellulose, light silicic acid anhydride, dextrin, carboxymethyl starch, gelatin, synthetic aluminum silicate, magnesium aluminometasilicate, magnesium oxide, calcium phosphate, calcium carbonate, calcium sulfate, and the like.

Preferred examples of the lubricant include, but not limited to, stearic acid, magnesium stearate, calcium stearate, talc, waxes, DL-leucine, sodium lauryl sulfate, magnesium lauryl sulfate, macrogol, Aerosil (possibly an antistatic agent), and the like.

Preferred examples of the binder include, but not limited to, gelatin, pullulan, hydroxypropyl methylcellulose (HPMC), hydroxypropylcellulose (HPC), methyl cellulose (MC), crystalline cellulose, polyvinylpyrrolidone (PVP), macrogol, gum arabic, dextran, polyvinyl alcohol (PVA), starch glue, and the like.

Preferred examples of the disintegrant include, but not limited to, carboxymethyl-cellulose, carboxymethyl-cellulose calcium, low-substituted hydroxypropylcellulose, cross-linked polyvinylpyrrolidone, carmellose sodium, croscarmellose sodium, carboxymethyl starch sodium, cation-exchange resins, partially pregelatinized starch, corn starch, and the like.

Examples of the thickener include, but not limited to, natural gums, cellulose derivatives, polyacrylic acids, and the like.

As preferred examples of the coating agent, hydrophilic high molecular weight polymers are generally used, and among them, water-soluble polymers such as cellulose derivatives e.g., hydroxyalkyl cellulose and alkyl cellulose, enteric coating polymers, coating polymers which dissolved in a stomach, and the like are preferably used. Further, these may be used in a mixture of 2 or more kinds thereof.

As the enteric coating polymers, for example, hydroxyalkyl cellulose phthalate such as hydroxypropyl methylcellulose phthalate; hydroxyalkyl cellulose acetate succinate such as hydroxypropylmethyl cellulose acetate succinate; carboxyalkyl cellulose such as carboxymethyl ethyl cellulose; cellulose acetate phthalate; a copolymer of ethyl acrylate and methacrylic acid, such as methacrylate copolymer L-100-55; a copolymer of methyl methacrylate and methacrylic acid such as methacrylate copolymer L and methacrylate copolymer S, or the like are preferably used.

As the polymers that dissolve in the stomach, for example, aminoalkyl methacrylate copolymer E; polyvinylacetal diethylaminoacetate, or the like are used.

In addition, copolymers containing a small amount of a quaternary ammonium group, for example, ethyl acrylate and methyl methacrylate such as a methacrylate copolymer RL and a methacrylate copolymer RS, and hydrophilic polymers such as carboxymethyl cellulose, a carboxyvinyl polymer, polyvinyl alcohol, gum arabic, sodium alginate, propylene glycol alginate, agar, gelatin and chitosan are used.

Preferred examples of the preservative include, but not limited to, paraoxybenzoate esters, chlorobutanol, benzyl alcohol, phenethyl alcohol, dehydroacetic acid, sorbic acid, and the like.

Preferred examples of the antioxidant include, but not limited to, sulphite, ascorbic acid and an alkali metal salt or alkaline earth metal salt thereof, and the like.

Preferred examples of the colorant include, but not limited to, synthetic colorants (for example, sunset yellow, its aluminum lake, and the like), yellow ferric oxide (Iron Oxide Yellow), ferric oxide (Iron Oxide Red), riboflavin, riboflavin organic acid esters (for example, riboflavin butyrate), riboflavin phosphate or an alkali metal salt or alkaline earth metal salt thereof, phenolphthalein, titanium oxide, and the like. Examples of the light-shielding agent include titanium oxide, and the like.

Preferred examples of the sweetening agent include, but not limited to, xylitol, saccharin sodium, glycyrrhizinic acid, dipotassium glycyrrhizinate, stevia extract, aspartame, and the like.

However, the total blending amount of the additives other than the surfactants is not such amount that gives effect for improving the granulatability of the low water-soluble compound. Preferably, the content of other additives is about 80% by weight or less with respect to the total granulated product, more preferably about 10 to 70% by weight, and particularly preferably about 20 to about 60% by weight.

The granulated product of the present invention can be prepared by adding a surfactant at a weight ratio of the surfactant to the low water-soluble compound of about 0.001 to about 2. The surfactant is preferably at a weight ratio of the surfactant to the low water-soluble compound of about 0.001 to less than 1, more preferably about 0.001 to less than 0.1, and particularly about 0.005 to about 0.05.

The granulated product can be prepared by a common method (for example, 10^{th} Edition, General Provision of Preparation, Japanese Pharmacopoeia). Specifically, (granulated) powdered drugs, fine granules, or granules can be produced by adding an excipient (for example, lactose, saccharose, glucose, starch, sucrose, crystalline cellulose, licorice powder, mannitol, sodium hydrogen carbonate, calcium phosphate, calcium sulfate, and the like), a disintegrant (for example, starch, calcium carbonate, and the like), and the like, if necessary, in addition to the low water-soluble compounds and the surfactant are added and mixed, a binder solution dissolved in an aqueous solvent (for example, water, water/ethanol, water/isopropanol, water/acetone, and the like) is added thereto, and the mixture is kneaded and compressed for granulation, or by putting a mixed powder containing low water-soluble compounds, a surfactant, and other additives into a granulator (for example, a tumbling granulator, a fluidized bed granulator, and the like) and spraying a binder solution dissolved in an aqueous solvent thereon to coat the mixed powder. The concentration of the binder in the binder solution may be about 3 to about 15% by weight. Further, the amount of the binder to be used may be one such that the weight ratio of the binder to the low water-soluble compound is about 2 to about 5%.

Alternatively, in each of the above-described methods, instead of mixing the low water-soluble compounds and the surfactant before the granulation, a surfactant may be blended by incorporating the surfactant into the binder liquid during the granulation. Particularly, a method wherein fluidized bed granulation is carried out with a binder solution incorporated with a surfactant is preferably carried out. In the case where the binder solution contains a surfactant, the concentration of the surfactant in the solution is not particularly limited as long as the weight ratio of the surfactant to the low water-soluble compound is about 0.001 to about 2 in the obtained granulated product, but it is preferably about 1 to about 1,000 mmol/L, and more preferably about 10 to about 100 mmol/L.

The granulated product of the present invention can be molded to a formulation which is favorably used for tablets, capsules, etc., for example, by a per se known means (for example, the method as described in 10^{th} Edition, General Provision of Preparation, Japanese Pharmacopoeia).

For example, in the case of a tablet, it can be prepared by tableting the mixture obtained by adding a lubricant (for example, magnesium stearate, stearic acid, calcium stearate, purified talc, and the like) to the obtained granulated product as described above. Further, in the case of a capsule, if necessary, thus obtained granulated product may be filled into a capsule made of gelatin, hydroxypropyl methylcellulose, or the like, along with an excipient (for example, lactose, saccharose, glucose, starch, sucrose, crystalline cellulose, licorice powder, mannitol, sodium hydrogen carbonate, calcium phosphate, calcium sulfate, and the like).

A tablet, a granule or a fine granule may be coated by means of a per se known method for the purpose of taste masking, enteric property or persistence. As the coating agent, for example, hydroxypropyl methylcellulose, ethyl cellulose, hydroxymethyl cellulose, hydroxypropylcellulose, polyoxyethylene glycol, Tween 80, Pluronic F68, cellulose acetate phthalate, hydroxypropylmethyl cellulose phthalate, hydroxypropylmethyl cellulose acetate succinate, Eudragit (manufactured by Röhm & Haas GmbH, West Germany, methacrylic acid/acrylic acid copolymers), and dyes such as titanium oxide, iron oxide red, and the like are used.

In the case where formulations such as a tablet, a capsule, a powdered drug, a granule, a fine granule tablet, and the like are made to preparations, the surface thereof may be further film-coated. In the case of the film-coating, light stability can be improved by the addition of a shielding agent, or the like. However, in this case, when a shielding agent such titanium oxide, and the like is added, firstly carrying out film coating without the shielding agent and then carrying out film coating with the shielding agent would yield better results.

Hereinbelow, the present invention will be described in detail with reference to Examples, but Examples should not be construed as limiting the present invention.

### EXAMPLES

### Example 1

Into a fluidized bed granulator (FD-5S manufactured by Powrex Corporation), 2,000 g of Compound X (3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepine-3-yl]acetyl]aminophenyl]propionic acid), 824.8 g of lactose and 711.2 g of crystalline cellulose were uniformly mixed, and then 40 g of sodium lauryl sulfate, 200 g of povidone (PVP) and 4 g of yellow ferric oxide were dissolved therein, and the resulting aqueous solution was sprayed and granulated, and then the resulting product was dried in the fluidized bed granulator. The obtained granulated product was pulverized using a power mill (P-3 manufactured by Showa Kagaku) with a punching size: 1.5 mmφ to obtain sized powders. To 3,402 g of the obtained sized powders, 180 g of croscarmellose sodium and 18 g of magnesium stearate were added and mixed using a tumbler mixer (TM-15S, manufactured by Showa Kagaku) to obtain granules to be tableted. The obtained granules were tableted using a rotary tableting machine (12HUK AW manufactured by Kikusui Seisakusho Co.) with a pestle of 14.0 x 8.0 mmφ (tableting pressure: 8 kN/pestle) to obtain 90,000 tablets each weighing 400 mg. The tablets having theoretical composition as described in Table 1 were obtained, respectively.

**Table 1**

| Composition | Comp. Ex. | Ex. 1 | Ex. 2 |
|---|---|---|---|
| Compound X | 200.00 | 200.00 | 200.00 |
| Lactose | 86.48 | 82.48 | 82.48 |
| Crystalline cellulose (PH-302) | 71.12 | 71.12 | 71.12 |
| Povidone | 20.00 | 20.00 | 20.00 |
| Yellow ferric oxide | 0.40 | 0.40 | 0.40 |
| Polysorbate 80 (Tween80) | - | 4.00 | - |
| Sodium lauryl sulfate (SLS) | - | - | 4.00 |
| Croscarmellose sodium | 20.00 | 20.00 | 20.00 |
| Magnesium stearate | 2.00 | 2.00 | 2.00 |
| Total (unit: mg) | 400.00 | 400.00 | 400.00 |

### Test Example 1 Wettability to binder

The bulk molded product of Compound X used in Example 1, and Compound X, lactose and crystalline cellulose in Table 1 (this mixture being referred to as a granulation component) were examined on the effect of the surfactant on the wettability to a binder (10% PVP) solution for the molded product. As a binding liquid, a solution of a binder alone (A), a solution of binder + a 1% polysorbate 80 (B), and a solution of a binder + 1% sodium lauryl sulfate (C) were used, and contact angle thereof were measured by using the following method.

About 300 mg of the bulk or the granulation component was weighed, and compression molded using a plain mortar and pestle of 10.0 mmφ by an autograph (manufactured by Shimadzu Co.) under the conditions of a tableting pressure of 10 kN/pestle and a tableting speed of 10 mm/min, thus to obtain a bulk molded product or a molded product of the granulation components. Onto the flat portion of each of the molded products, about 10 µL of the above-described binder liquid was dropped using a Microsyringe (manufactured by ITO SEISAKUSHO CO., LTD.), and the solid and liquid interface immediately after dropping was photographed by a digital microscope (Keyence). After photographying, using an accompanying software, the angle between the liquid surface and the solid surface was measured and referred to as an angle of repose. The results are shown in Table 2. Incorporating the surfactant into the binder liquid remarkably improved the wettability of Compound X to the binder liquid.

**Table 2**

| Binder liquid used | (A) | (B) | (C) |
|---|---|---|---|
| Contact angle (°) with respect to bulk molded product | 56 | 33 | 28 |
| Contact angle (°) with respect to granulation component molded product | 80 | 36 | 25 |

### Test Example 2 Characteristics of granulated powder of Compound X

The characteristics of the granulated powders before tableting each of the tablets obtained in Example 1 (rough specific volume, specific filling volume, particle size distribution, angle of repose) were examined by the following methods.

Rough specific volume: 50 g of granulated powders are weighed and put into a 200 mL-measuring cylinder using a funnel. The upper surface of the powders is flattened using a brush, and at this time the scale is recorded. The value obtained by subtracting 50 g from the scale is referred to as a rough specific volume (mL/g).

Specific filling volume: After measuring the rough specific volume, the measuring cylinder incorporated with a sample is repeatedly dropped from the height of 5 cm, and at a time point when the upper surface of the powders becomes uniform, the scale is recorded. The value obtained by subtracting 50 g from the scale is referred to as a specific filling volume (mL/g).

Particle size distribution: 50 g of the granulated powders are weighed, put into the top sieve of the set having the upper part equipped with the sieves of 16 M, 30 M, 42 M, 60 M and 100 M in this order, and vigorously shaken 300 times. After settling for 1 to 2 minutes, the sample remaining on each of the sieves or the sample passing through the sieves is weighed. From the ratio of each fraction to the total weight, a particle size distribution (%) is obtained.

Angle of repose: 20 g of the granulated product is supplied from the upper part of the funnel using a Powder Tester (Type PT-E) (manufactured by Hosokawa Micron Co., Ltd.), and is subjected to moderate vibration by means of a vibrator, and the granulated product is dropped. After completely dropping the powders, the angle between the powder layer and the horizontal surface is referred to an angle of repose.

The results are shown in Table 3. By blending the surfactant, the particle growth is remarkably improved, thus flowability being also improved.

**Table 3**

| | Comp. Ex. | Ex. 1 | Ex. 2 |
|---|---|---|---|
| Rough specific volume (mL/g) | 2.20 | 2.42 | 2.60 |
| Specific filling volume (mL/g) | 1.84 | 1.92 | 2.00 |
| Not passed through 16 M (> 1000 µm) | 0.0 | 0.0 | 0.0 |
| Not passed through 30 M (> 500 µm) | 1.4 | 0.0 | 2.3 |
| Not passed through 42 M (> 355 µm) | 3.3 | 0.2 | 24.2 |
| Not passed through 60 M (> 250 µm) | 5.8 | 5.1 | 29.6 |
| Not passed through 100 M (> 150 µm) | 19.1 | 46.4 | 27.1 |
| Passed through 100 M (< 150 µm) | 70.3 | 48.3 | 16.8 |
| Angle of repose (°) | 55 | 35 | 32 |

### Industrial Applicability

According to the method of the present invention, the granulatability of the composition can be improved by adding a small amount of surfactant, without adding a large amount of an excipient, etc, and thus it becomes possible to prepare a tablet, a capsule, or the like in the formulation having a high proportion of the low water-soluble compounds without enlarging the solid composition.

The present invention claims the priority of Japanese Patent Application No. 2003-430169 (filed on December 25, 2003), which is incorporated by reference herein by its entirety.

## Claims

1. A pharmaceutical granulated product having improved granulatability, which contains a pharmaceutical compound with poor wettability and a surfactant.

2. A granulated product, wherein the product contains a compound with poor wettability and a surfactant, and at least about 35% by weight with respect to the total weight of the product does not pass through a 100-mesh sieve.

3. The granulated product according to claim 2, wherein the weight ratio of the compound and the surfactant is 1 : about 0.001 to about 2.

4. The granulated product according to claim 3, wherein the weight ratio is 1 : about 0.001 to less than 1.

5. The granulated product according to claim 3, wherein the weight ratio is 1 : about 0.001 to less than 0.1.

6. The granulated product according to claim 3, wherein the weight ratio is 1 : about 0.005 to about 0.05.

7. The granulated product according to claim 2, wherein the ratio of the compound with respect to the total granulated product is about 20% by weight or more.

8. The granulated product according to claim 2, wherein the compound is a pharmaceutical compound.

9. A molded product made by molding the granulated product according to any one of claims 2 to 8.

10. A method for improving granulatability of a pharmaceutical composition containing a pharmaceutical compound with poor wettability, which comprises adding a surfactant before or during the granulation.

11. A method for preparing a granulated product containing a compound with poor wettability, having improved granulatability, which comprises adding a surfactant in the weight ratio of about 0.001 to about 2 with respect to the compound before or during the granulation.

12. The method according to claim 11, wherein a granulated product is obtained in which at least about 35% by weight with respect to the total weight of the product does not pass through a 100-mesh sieve.

13. The method according to claim 11, which involves wet granulation in a binder solution containing a surfactant.

14. The method according to claim 13, wherein the concentration of the surfactant in the binder solution is about 1 to about 1,000 mmol/L.

15. The method according to claim 13, wherein the concentration of the surfactant in the binder solution is about 10 to about 100 mmol/L.

16. The method according to claim 11, wherein the compound is a pharmaceutical compound.

17. A method for preparing a molded product, comprising molding the granulated product which is obtained by the method according to any one of claims 11 to 16.

18. A use of a surfactant for improving granulatability during the granulation of a pharmaceutical composition.

19. An agent for improving granulatability during the granulation of a pharmaceutical composition, which contains a surfactant.
